# FASCICULE DE BREVET EUROPEEN

(11) **EP 1 427 387 B1**
(45) Date de publication et mention de la délivrance du brevet: **20.02.2008**
(21) Numéro de dépôt: 01934081.9
(22) Date de dépôt: 10.05.2001
(51) Int. Cl.: A61K 8/97, A61Q 19/08

(54) **PRODUIT COSMETIQUE POUR LES SOINS DE LA PEAU**
KOSMETISCHES HAUTPFLEGEMITTEL
COSMETIC PRODUCT SUITABLE IN PARTICULAR FOR SKIN CARE

(30) Priorité: 10.05.2000 FR 0005942
(43) Date de publication de la demande: 16.06.2004
(73) Titulaire: Fauvel, Michel, 72000 Le Mans (FR); Drouet, Marcelle, 72000 Le Mans (FR)
(72) Inventeur: Fauvel, Michel, 72000 Le Mans (FR); Drouet, Marcelle, 72000 Le Mans (FR)
(86) Numéro de dépôt international: PCT/FR2001/001417
(87) Numéro de publication internationale: WO 2001/085127

(56) Documents cités:
- EP-A- 0 085 589
- DE-A- 4 130 324
- DE-A- 19 624 476
- FR-A- 2 744 915
- DATABASE WPI Week 199828 Derwent Publications Ltd., London, GB; AN 1998-320407 XP002157112 & RU 209 603 C (AS USSR CHEM BIOLOG ASSOC STOCK CO)
- F.CORMIER ET.AL.: "Growth Kinetics of Vitis vinifera Cell Suspension Cultures: I. Shake Flask Cultures" BIOTECHNOLOGY AND BIOENGINEERING, vol. 47, 1995, pages 131-138, XP002066125
- A.DECENDIT ET.AL.: "Production, purification et activité biologique des picéides (stilbènes) extraits de cultures cellulaires de Vitis vinifera L." BULL.SOC.PHARM.BORDEAUX, vol. 136, 1997, pages 7-18, XP002929668

## Description

L'invention se rapporte aux produits d'hygiène et de beauté.

Elle concerne plus particulièrement un produit cosmétique convenant notamment aux soins de la peau.

On connaît déjà de nombreux produits cosmétiques qui comprennent un ou plusieurs principes actifs en mélange avec un véhicule physiologiquement acceptable, convenant à une application topique.

Ces principes actifs peuvent être d'origine naturelle ou synthétique et sont incorporés dans le véhicule pour former un produit qui se présente habituellement sous la forme d'une crème, d'un lait, d'une lotion, d'un gel, d'une huile, etc.

Lorsque le produit cosmétique est ainsi appliqué sur la peau, les principes actifs viennent alors exercer leurs propriétés sur la peau.

De tels principes actifs sont choisis, en fonction de leurs propriétés, pour, par exemple, retarder le vieillissement de la peau, apaiser ou hydrater la peau, la protéger contre les agressions extérieures, la régénérer, etc.

Ainsi, les principes actifs doivent être choisis en fonction des propriétés à exercer sur la peau.

On sait par ailleurs que certains produits, tels que les polyphénols, sont doués de propriétés anti-radicalaires et anti-oxydantes qui leur permettent d'exercer une action avantageuse sur la peau.

Malheureusement, il a été jusqu'à présent difficile de pouvoir trouver des principes actifs susceptibles de libérer, dans des conditions choisies et contrôlées, des catégories spécifiques de polyphénols, en fonction de l'application souhaitée.

L'invention a notamment pour but de surmonter de tels inconvénients.

Elle propose à cet effet un produit cosmétique convenant notamment au traitement de la peau, lequel comprend en tant que principe actif des cellules fraîches de vigne à teneur en polyphénols, ces cellules étant en mélange avec un véhicule physiologiquement acceptable et convenant à une application topique.

Par l'expression "cellules fraîches", on entend désigner ici des cellules vivantes issues de culture.

Il a été établi que les cellules de vigne cultivées in vitro ont une composante majeure en polyphénols.

Ces polyphénols se divisent en sept grands groupes présents dans les cellules, à savoir : les catéchines, les épicatéchines, l'épicatéchine gallate, l'épigallocatéchine-SH, la catéchine-SH, l'épicatéchine-SH et l'épicatéchine gallate-SH.

Ces cellules de vigne ont pour avantage de disposer de principes actifs très performants provenant directement du végétal, sans qu'il soit nécessaire de procéder à une extraction d'origine chimique par solvant.

Il en résulte une disponibilité globale de la cellule qui regroupe tous les polyphénols à l'état naturel.

On peut ainsi incorporer les cellules vivantes de vigne, dès leur croissance terminée, directement dans des produits cosmétiques, tels que crèmes, laits, lotions, gels, huiles, etc. Elles permettent ainsi d'exercer directement leur action bénéfique sur la peau soit seules, soit en complément d'un ou de plusieurs autres principes actifs également contenus dans le produit cosmétique.

A l'heure actuelle, nombre de polyphénols sont extraits de végétaux différents pour des applications spécifiques.

L'originalité de l'invention consiste, au travers des cellules de vigne, à libérer d'une manière biologiquement naturelle tous les polyphénols définis ci-dessus et présents dans les cellules.

L'activité qui en résulte permet, au travers de chaque catégorie de polyphénols, d'obtenir une crème, un lait, une lotion, un gel, une huile, etc répondant à des critères d'utilisation spécifique, et ceci grâce à la même origine cellulaire.

Les cellules fraîches de vigne proviennent avantageusement de plantes du genre Vitis (vigne), et en particulier de l'espèce Vitis vinifera.

Ces cellules fraîches peuvent provenir d'une vigne cultivée, quel que soit le cépage, ou encore d'une vigne sauvage, quel qu'en soit le pays d'origine.

Selon une autre caractéristique de l'invention, les cellules fraîches de vigne proviennent d'une culture menée in vitro dans des conditions choisies pour obtenir une production contrôlée des polyphénols.

Selon encore une autre caractéristique de l'invention, les cellules fraîches de vigne sont incorporées directement dans le véhicule du produit.

Dans une première application de l'invention, le produit cosmétique est réalisé sous la forme d'une crème qui comprend essentiellement les phases suivantes :
phase aqueuse A contenant de l'eau,
phase grasse B contenant un émulsionnant,
phase C contenant un conservateur, et
phase D contenant les cellules fraîches de vigne et éventuellement d'autres produits actifs sensibles.

Dans une autre application de l'invention, le produit cosmétique est réalisé sous la forme d'un lait qui comprend essentiellement les phases suivantes :
phase aqueuse A contenant de l'eau,
phase grasse B contenant un émulsionnant et un émollient,
phase C contenant un conservateur, et
phase D contenant les cellules fraîches de vigne et éventuellement d'autres produits actifs sensibles.

Dans une autre application de l'invention, le produit cosmétique est réalisé sous la forme d'une lotion qui comprend une phase aqueuse A contenant de l'eau, un glycol, un conservateur, les cellules fraîches de vigne et éventuellement d'autres produits actifs sensibles.

Il est possible de réaliser le produit cosmétique sous d'autres formes, en particulier sous la forme d'un gel, d'une huile ou d'un produit capillaire.

On décrira maintenant l'invention en référence aux exemples suivants, donnés uniquement à titre illustratif.

### Exemple 1

### Préparation de cellules de vigne

Dans cet exemple, on part de souches de cellules de vigne (Vitis vinifera) provenant de cépages riches en polyphénols, ces cellules provenant de pétioles ou de jeunes tiges prélevées en champs.

On suit le protocole général, indiqué ci-dessous, pour la production de métabolites secondaires par des cultures végétales in vitro.

Des suspensions cellulaires de vigne sont repiquées au laboratoire dans des fioles d'Erlenmeyer, sous conditions stériles, chaque semaine dans un milieu de culture appelé "milieu d'entretien" permettant la survie et la croissance des cellules. Ce milieu de culture apporte des éléments minéraux, des vitamines, des phytohormones et du saccharose, en quantités connues et exactement pesées au laboratoire.

Les cellules en suspension se présentent sous forme de petits agrégats de quelques cellules à quelques dizaines de cellules, la dimension d'une cellule étant d'environ 50 micromètres.

Les cellules sont cultivées dans un bio-réacteur en utilisant un milieu de culture stimulant la production et l'accumulation des polyphénols. Ce milieu, appelé "milieu de production" est identique au milieu d'entretien, sauf que sa teneur en saccharose est multipliée par trois et que ces teneurs en sulfate d'ammonium et phosphate de sodium sont multipliées par deux. D'autres modifications de ce milieu sont possibles pour améliorer encore la production.

Les conditions de culture dans le bio-réacteur sont les suivantes :
- conditions de cultures stériles,
- procédés du type en discontinu,
- aération de 0,1 à 0,2 vvm (où vvm signifie volume d'air/volume du milieu/minute),
- agitation de 70 à 100 tours/minute avec une turbine à pales inclinées ou une turbine classique à pales droites du type Rushton,
- rapport quantité d'inoculum, milieu de production : 1/5 à 1/8,
- durée de la culture : 14-18 jours,
- température : 25° C,
- volume du bio-réacteur : 2 litres à 400 litres.

Après la culture, les cellules sont filtrées pour éliminer le milieu de culture résiduel et rincées à l'eau distillée froide. La biomasse obtenue est d'environ 350 grammes de matière fraîche par litre de culture, équivalent à 25 grammes de matière sèche après lyophilisation. La teneur en polyphénols est d'environ 1% par rapport à la matière. Ces polyphénols sont stockés dans les vacuoles des cellules de vigne.

De telles cellules peuvent être utilisées directement pour former des produits cosmétiques.

On donnera ci-après quelques exemples, non limitatifs, de tels produits cosmétiques.

### Exemple 2 : Crème de jour

Phase aqueuse A : eau associée à un acide pyrrolidone carboxylique (PCA),
Phase grasse B : émulsionnant + triglycéride ou pidolate,
Phase C : conservateur,
Phase D : produit actif sensible : parfums, extraits de plantes, et cellules fraîches de vigne.

### Exemple 3 Crème de nuit

Phase aqueuse A : eau associée à un pidolate,
Phase grasse B : émulsionnant + émollient,
Phase C : conservateur + biosaccharide,
Phase D : produit actif sensible : parfums, glycolysats végétaux, et cellules fraîches de vigne.

### Exemple 4 : Crème super restructurante

Phase aqueuse A : eau associée à un PCA hydratant,
Phase grasse B : émulsionnant + émollient + triglycérides,
Phase C : conservateur,
Phase D : produit actif sensible : parfums, A.H.A. (a-hydroxyde acide) , et cellules fraîches de vigne.

### Exemple 5 : Crème contour yeux

Phase aqueuse A : eau associée à un PCA hydratant longue durée,
Phase grasse B : émulsionnant,
Phase C : conservateur,
Phase D : produit actif sensible : parfums, huiles essentielles, et cellules fraîches de vigne.

### Exemple 6 : Crème pour les mains

Phase aqueuse A : eau associée à un pidolate anti-bactérien,
Phase grasse B : émulsionnant + émollient,
Phase C : conservateur + hydratant,
Phase D : produit actif sensible : parfums, hydrolysat, végétal, et cellules fraîches de vigne.

### Exemple 7 Crème pour les pieds

Phase aqueuse A : eau associée à un pidolate hydratant anti-bactérien,
Phase grasse B : émulsionnant + émollient,
Phase C : conservateur,
Phase D : produit actif sensible : parfums, huile végétale, et cellules fraîches de vigne.

### Exemple 8 Crème solaire

Phase aqueuse A : eau associée à un pidolate hydratant,
Phase grasse B : émulsionnant + émollient,
Phase C : conservateur + protecteur minéral + hydratant filmogène,
Phase D : produit actif sensible : parfum, huile essentielle, et cellules fraîches de vigne.

### Exemple 9 : Lait corporel

Phase aqueuse A : eau associée à un pidolate hydratant,
Phase grasse B : émulsionnant + émollient,
Phase C : conservateur + hydratant,
Phase D : produit actif sensible : parfum, A.H.A., huile végétale, et cellules fraîches de vigne.

### Exemple 10 : Lait démaquillant

Phase aqueuse A : eau associée à un hydratant,
Phase grasse B : émulsionnant + émollient,
Phase C : conservateur,
Phase D : produit actif sensible : parfum, hydrolysat végétal, et cellules fraîches de vigne.

### Exemple 11 : Lotion démaquillante, lotion tonifiante

Phase aqueuse A : eau associée à une eau florale, propylène glycol, conservateur, produit actif sensible : huile essentielle, extrait de fruit et cellules fraîches de vigne.

Dans les exemples ci-dessus, concernant des crèmes ou des laits corporels, les différentes phases A, B, C et D sont mélangées de manière habituelle, ainsi qu'il est bien connu de l'homme de l'art dans cette technique.

Les proportions des différentes phases peuvent varier entre elles, et de même, on peut varier le pourcentage des produits actifs sensibles contenus dans le produit cosmétique, et cela également en fonction des applications souhaitées.

Dans le cas des lotions, le produit cosmétique ne contient qu'une seule phase aqueuse et les différents constituants sont mélangés en une seule phase, également d'une manière en soi connue dans cette technique.

La proportion des cellules de vigne dans le produit cosmétique dépend de la nature de ce produit et de celle des soins à prodiguer. Elle est généralement comprise entre 3 et 10% en poids et peut atteindre des valeurs plus élevées, par exemple de 25%, pour des soins intensifs.

Il va de soi que les exemples donnés ci-dessus ne sont que des exemples de réalisation et que de nombreuses variantes entrent également dans le cadre de l'invention.

Ainsi il est possible aussi de réaliser le produit cosmétique sous d'autres formes, par exemple sous celle d'un gel ou d'une huile.

Egalement, le produit cosmétique peut être réalisé sous la forme d'un produit de maquillage (rouge à lèvres, poudre, fond de teint, etc) ou d'un produit capillaire.

Dans tous les cas, on obtient un produit cosmétique qui contient des cellules fraîches de vigne ayant été cultivées dans des conditions choisies pour contenir des catégories sélectionnées de polyphénols en fonction des applications choisies.

## Revendications

1. L'utilisation pour les soins de la peau, d'un produit cosmétique **caractérisé en ce qu'**il utilise en tant que principe actif des cellules fraîches de vigne déjà connues à teneur en Polyphénols, les dites cellules étant en mélange avec un véhicule physiologiquement acceptable et convenant à une application topique.

2. L'utilisation pour les soins de la peau d'un produit cosmétique selon la revendication 1, **caractérisé en ce que** les cellules fraîches de vigne utilisées proviennent de plantes du genre Vitis (vigne) et en particulier de l'espèce Vitis Vinifera.

3. L'utilisation pour les soins de la peau d'un produit cosmétique selon l'une des revendications 1 et 2, **caractérisé en ce que** les cellules fraîches de vigne proviennent d'une vigne cultivée.

4. L'utilisation pour les soins de la peau d'un produit cosmétique selon l'une des revendications 1 et 2, **caractérisé en ce que** les cellules-fraîches de vigne proviennent d'une vigne sauvage.

5. L'utilisation pour les soins de la peau d'un produit cosmétique selon l'une des revendications 1 à 4, **caractérisé en ce que** les cellules fraîches de vigne proviennent d'une culture menée in vitro, dans des conditions choisies pour obtenir une production contrôlée de polyphénols.

6. L'utilisation pour les soins de la peau d'un produit cosmétique selon l'une des revendications 1 à 5, **caractérisé en ce que** les cellules fraîches de vigne sont incorporées dans le véhicule du dit produit.

7. L'utilisation pour les soins de la peau d'un produit cosmétique selon l'une des revendications 1 à 6, **caractérisé en ce qu'**il est réalisé sous la forme d'une crème, d'un lait, d'une lotion, d'un gel, d'une huile ou d'un produit de maquillage.

## Claims

1. The use for skin care of a cosmetic product, characteristic in that it uses as an active ingredient fresh vine cells, already recognised for their content in Polyphenols, the aforementioned cells being combined with a medium that is physiologically acceptable and suitable for a topical *application*.

2. The use for skin care of a cosmetic product in accordance with claim 1, characteristic in that the fresh vine cells being used come from plants of the genus Vitis (vine) and in particular from the species Vitis Vinifera.

3. The use for skin care of a cosmetic product in accordance with one of the claims 1 and 2, characteristic in that the fresh vine cells come from a cultivated vine.

4. The use for skin care of a cosmetic product in accordance with one of the claims 1 and 2, characteristic in that the fresh vine cells come from a wild vine.

5. The use for skin care of a cosmetic product in accordance with one of the claims 1 to 4, characteristic in that the fresh vine cells come from a culture conducted in vitro, in conditions chosen for obtaining a controlled production of polyphenols.

6. The use for skin care of a cosmetic product in accordance with one of the claims 1 to 5, characteristic in that the fresh vine cells are incorporated into the medium of the aforementioned product.

7. The use for skin care of a cosmetic product in accordance with one of the claims 1 to 6, characteristic in that it is produced in the form of a cream, a milk, a lotion, a gel, an oil or a make-up product.

## Patentansprüche

1. Die Anwendung der Hautpflegeprodukte, eines Kosmetikproduktes, das durch den verwendeten Wirkstoff von bereits bekannten, polyphenolhaltigen Frischzellen eines Weinstocks charakterisiert wird, werden auch Zellen genannt, die mit einem physiologisch akzeptablem Bindemittel vermischt werden und die zu einer topischen Anwendung passen.

2. Die Anwendung der Hautpflegeprodukte eines Kosmetikproduktes gemäß Forderung 1 wird dadurch charakterisiert, dass die angewandten Frischzellen eines Weinstocks und von Pflanzen des Typs Vitis (Weinstock) und vor allem aus dem Raum Vitis Vinifera stammen.

3. Die Anwendung der Hautpflegeprodukte eines Kosmetikproduktes gemäß einer der Forderungen 1 oder 2 wird durch die Frischzellen eines Weinstocks, die von einem gezüchteten (kultivierten) Weinstock stammen, charakterisiert.

4. Die Anwendung der Hautpflegeprodukte eines Kosmetikproduktes gemäß einer der Forderungen 1 oder 2 wird durch die Frischzellen eines Weinstocks, die von einem wilden Weinstock stammen, charakterisiert.

5. Die Anwendung der Hautpflegeprodukte eines Kosmetikproduktes gemäß einer der Forderungen 1 bis 4 wird durch die Frischzellen eines Weinstocks, die aus einer in vitro geführten Kultur stammen sowie aus ausgewählten Bedingungen, um eine polyphenolkontrollierende Produktion zu erhalten, charakterisiert.

6. Die Anwendung der Hautpflegeprodukte eines Kosmetikproduktes gemäß einer der Forderungen 1 bis 5 wird durch das charakterisiert, was die Frischzellen eines Weinstocks im Bindemittel des besagten Produkts aufnehmen / vereinigen.

7. Die Anwendung der Hautpflegeprodukte eines Kosmetikproduktes gemäß einer der Forderungen 1 bis 6 wird durch das charakterisiert, was in Form einer Creme, einer Milch, einer Lotion, eines Gels, eines Öls oder eines Schminkproduktes realisiert wird.
